(19) **Europäisches Patentamt European Patent Office Office européen des brevets**

(11) **EP 4 454 567 B1**

(12) **EUROPEAN PATENT SPECIFICATION**

(45) Date of publication and mention of the grant of the patent:
**29.10.2025 Bulletin 2025/44**

(21) Application number: **23195100.5**

(22) Date of filing: **04.09.2023**

(51) International Patent Classification (IPC):
**A61B 6/03** (2006.01)    **A61B 6/00** (2024.01)

(52) Cooperative Patent Classification (CPC):
**A61B 6/032; A61B 6/405; A61B 6/486; A61B 6/5264; A61B 6/527; A61B 6/541; A61B 6/542**

(54) **REDUCING AN X-RAY DOSE APPLIED ON A SUBJECT DURING A RESPIRATION-CORRELATED COMPUTED TOMOGRAPHY SCAN**

**REDUZIERUNG EINER AUF EINE PERSON AUFGEBRACHTEN RÖNTGENDOSIS WÄHREND EINES ATMUNGSKORRELIERTEN COMPUTERTOMOGRAFIESCANS**

**RÉDUCTION D'UNE DOSE DE RAYONS X APPLIQUÉE À UN SUJET PENDANT UN SCAN DE TOMODENSITOMÉTRIE CORRÉLÉ À LA RESPIRATION**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**30.10.2024 Bulletin 2024/44**

(73) Proprietor: **Siemens Healthineers AG 91301 Forchheim (DE)**

(72) Inventors:
• **Hofmann, Christian 91058 Erlangen (DE)**
• **Vornehm, Marc 91054 Erlangen (DE)**

(74) Representative: **Siemens Healthineers Patent Attorneys Postfach 22 16 34 80506 München (DE)**

(56) References cited:
**DE-A1- 102011 079 496**

• **R. WERNERT. SENTKERF. MADESTAT. GAUERC. HOFMANN: "Intelligent 4D CT sequence scanning (i4DCT): Concept and performance Evaluation", MED. PHYS., vol. 46, no. 8, August 2019 (2019-08-01), pages 3462 - 3474, XP093105630**

**Description**

The invention relates to a computer-implemented method for reducing an X-ray dose applied on a subject during a respiration-correlated computed tomography scan and a corresponding computer program computer-readable storage medium, and computed tomography system.

Independent of the grammatical term usage, individuals with male, female or other gender identities are included within the term.

DE 10 2011 079 496 A1 discloses a method for generating combined tomographic emission and transmission images of a breathing and thereby cyclically moving patient.

Respiration-correlated computed tomography (CT) imaging, also referred to as four-dimensional (4D) CT imaging, is frequently used as basis for radiotherapy treatment planning. 4D CT imaging is a method for measuring anatomic motion induced by patient respiration. During CT acquisition, respiration of the patient is monitored and set in relation to the initiation time of the CT scan. Accordingly, reconstructed CT slices can be correlated to the breathing phase of the patient's respiratory cycle. By selecting corresponding slices correlated to different phases of the breathing cycle, CT images can be generated at multiple breathing phases.

4D CT image quality has traditionally been severely affected by image artefacts. This originates from the retrospective analysis and correlation of the breathing cycles with the CT scan. For example due to irregularities in a patient's breathing cycle, the acquired data coverage may be insufficient for reconstruction of image slices at particular breathing states. This might even lead to the necessity of repeating the CT scan and thus additional X-ray dose being applied. In order to tackle this problem, it has been proposed to fulfil a data sufficiency condition (DSC). The DSC demands that for each couch position of a desired scan range, projection data have been acquired for at least an entire breathing cycle of the patient. A corresponding method is described by R. Werner, T. Sentker, F. Madesta, T. Gauer, and C. Hofmann in "Intelligent 4D CT sequence scanning (i4DCT): Concept and performance Evaluation", Med. Phys. 46 (8), August 2019, p. 3462-3474. The intelligent 4D CT sequence scanning (i4DCT) is a method for respiration-correlated 4D CT scanning in which the start and end points (beam on and beam off events) of the data acquisition process at a given couch position are triggered by the patient's respiration. Each beam on period covers a full breathing cycle from end-inhalation to end-inhalation to ensure fulfilment of the DSC at each couch position. With this adaption, it can be ensured that all relevant breathing states of a patient's breathing cycle are recorded at each couch position.

While i4DCT has thus introduced a considerable advantage, it is still desirable to further improve 4D CT imaging. In particular, it would be advantageous to reduce the applied X-ray dose.

It is therefore an object of the invention to provide a way to improve 4D CT imaging, namely to provide a way to reduce an applied X-ray dose.

This object is met or exceeded by a method according to claim 1, a computer program according to claim 13, a computer-readable storage medium according to claim 14 and a computed tomography system according to claim 15. Further advantages and features result from the dependent claims, the description and the attached figures.

According to a first aspect of the disclosure, a computer-implemented method for reducing an X-ray dose applied on a subject during a respiration-correlated computed tomography (CT) scan is provided. The method comprises the following steps:

(a) during a beam-on period of the scan, determining the beginning of a data-redundant breathing phase;
(b) during the data-redundant breathing phase, applying a redundancy modulation to the applied X-ray dose such that the X-ray dose is reduced during the data-redundant breathing phase;
(c) determining the end of the data-redundant breathing phase;
(d) at the end of the data-redundant breathing phase, continuing the scan without the redundancy modulation of the X-ray dose.

Advantageously, the X-ray dose applied to the subject may be reduced via this method by taking into account data-redundant breathing phases and modulating the applied X-ray dose accordingly.

A subject may be a human being or an animal. For example, the subject may be a patient. The subject may be a patient that is subjected to radiation therapy, in particular radiation therapy that is monitored via respiration-correlated CT scanning.

The term "X-ray dose" is to be understood broadly in the context of this invention. It generally describes an X-ray radiation dose quantity that is absorbed by and/or sent towards a subject or a part of a subject. Typically, the dose may be absorbed by tissue of the subject. It can be a measure of radiation energy that incident radiation deposits in the subject's tissue. The dose can be measured in the SI unit "gray" (Gy) or in other units, such as the unit "Rad". Both, overall dose (absorbed by the subject in total) as well as a local dose (absorbed by a part of the subject, e.g. a scanned organ), can be relevant. The X-ray dose may be controlled by controlling the output of at least one X-ray source of a CT system. Controlling the X-ray source may, for example, comprise controlling the intensity of emitted X-ray radiation, the direction of the emitted X-ray radiation (in particular relative to the location of the subject), and/or a blocking of a radiation path of emitted X-ray radiation.

A beam-on period of the scan is a period in which at least one X-ray beam is switched on. Typically, during a CT scan, the subject is moved into different couch positions, and CT data are acquired at the different couch positions. The couch position describes the position of the subject in the direction of a CT system's gantry longitudinal axis. The position of the subject is typically adjusted by moving a movable patient table. The couch position may also be referred to as z-position. The time of data acquisition usually corresponds to a beam-on period, i.e. to the time that the X-ray beam used for data acquisition is switched on. Preferably, the beam-on periods of the CT scan may cover a full breathing cycle, respectively. A full breathing cycle may comprise a whole breathing process from one breathing state till the next periodic reoccurrence of the same breathing state. For example, a full breathing cycle may go from end-inhalation to end-inhalation. End-inhalation can be defined to be the breathing state at maximum inhalation, in particular just before the subject starts breathing out again. Preferably, in the context of this invention, a breathing cycle may be considered to begin and end at an end-inhalation state, in particular at two consecutive end-inhalation states.

The respiration-correlated computed tomography scan may be based on covering a full breathing cycle at each couch position. Preferably the breathing cycle may be covered from end-inhalation to end-inhalation. This can be a particularly suitable point in the breathing cycle to monitor the breathing cycle and adjust the CT scan accordingly. The start and end points (i.e., beam on and beam off events) of the data acquisition process at a given couch position may be triggered by a real-time analysis of the patient's respiration. Preferably, in between consecutive beam-on periods there may be at least one breathing cycle, during which the X-ray beam is switched off. In other words, in between consecutive beam-on periods there may be beam-off periods having a duration of at least one breathing cycle. The respiration-correlated computed tomography scan may in particular be based on intelligent 4D CT sequence scanning (i4DCT).

The term "breathing phase" is to be understood broadly in the context of this invention. It generally describes a phase, i.e. a part, of the breathing cycle. A breathing phase may, for example, be a part of the breathing cycle during which the subject inhales or a part during which the subject exhales or a part during which the subject pauses from both inhaling and exhaling, e.g. between an exhaling phase and an inhaling phase. However, any other kind of breathing phase may be determined as well, e.g. depending on the context.

A data-redundant breathing phase is in particular a breathing phase, during which no additional CT data can be acquired during a breathing cycle and/or at one couch position that contains any additional significant information with respect to data acquired during the rest of the breathing cycle at the current couch position. The beginning and end of the data-redundant breathing phase may be determined by monitoring the breathing of the subject. Determining the beginning and/or the end of the data-redundant breathing phase may be based on a reference breathing cycle. The reference breathing cycle may be specific for the subject that is currently examined by the CT scan. The reference breathing cycle may be determined based on data acquired before the beginning of the CT scan. Additionally or alternatively the reference breathing cycle may be determined based on data acquired during the CT scan. The reference breathing cycle may be updated during the CT scan. The updating may be based on monitoring the breathing of the subject during the scan. Updating the reference breathing cycle may be advantageous to counter drift effects of the monitoring or changes in the subject's breathing pattern. The redundancy modulation may be applied by reducing the X-ray output of at least on X-ray source of the CT system. Additionally and/or alternatively, the redundancy modulation may be applied by changing the direction of the X-ray output of at least on X-ray source of the CT system. Additionally and/or alternatively, the redundancy modulation may be applied by blocking at least part of the X-ray output of at least on X-ray source of the CT system. The term redundancy modulation is generally to be understood broadly in the context of this invention and it is in particular defined to provide a reduction in the applied X-ray dose. Optionally, a reduction of the X-ray dose may comprise a reduction of the X-ray dose to zero or nearly zero.

It has been found by the inventors that for many patients, at least during some cycles, a breathing cycle comprise breathing phases of up to several seconds where no significant respiratory motion takes place. This is typically the case in an end-exhalation state. The inventors have noticed that data acquired during such a breathing pause does not provide significant additional information or only very little additional information. Since respiration-correlated computed tomography aims to observe data for several breathing states (e.g. at maximum inhalation, at minimal inhalation and at breathing states in between these end points), data from such a breathing pause is often not actually needed to monitor all the required breathing states. Accordingly, instead of applying the data sufficiency condition by acquiring an entire breathing cycle at full dose, the X-ray dose can be reduced at redundant parts of the breathing cycle. Modulating the X-ray dose to be reduced can thus advantageously prevent the exposure of the subject to unnecessary ionizing radiation. Hence, the X-ray dose may be reduced when data redundancy allows for it. Thus, the dose may be reduced without significant restrictions to the full phase selectivity of a retrospective 4D CT reconstruction.

According to an embodiment, a breathing phase is determined to be data-redundant when it is estimated to provide no significant additional breathing states during the current breathing cycle and/or at the current couch position. For example, it may be provided that the breathing is determined to be data-redundant when a breathing pause after an exhalation phase is detected.

In the context of this invention, the term "breathing state" is to be understood broadly. It generally may describe any state of the subject's breathing. For example, maximum inhalation or minimum inhalation during a breathing cycle may be examples of breathing states. The breathing state may in particular be correlated or defined by the extension and contraction of the subject's lung. The breathing state may be defined by a surrogate parameter. The surrogate parameter may be related to the subject's breathing state. For example, an elevation of the subject's chest may be used to define the breathing state.

The term "significant additional breathing state" may be understood such that an additional breathing state is very close to at least one already measured breathing state during this breathing cycle and/or at the current couch position. The already measured breathing state may in particular be the previous breathing state. The term "very close" may be defined by a threshold difference. Additionally or alternatively, the term "very close" may be defined by a typical measurement accuracy and/or measurement signal noise.

According to an embodiment, a breathing phase is determined to be data-redundant, when a change of the breathing state over time is estimated to be below a threshold and/or when a variance of the breathing state is estimated to remain within a predetermined range. Advantageously the application of the redundancy modulation may thus start as soon as the breathing state remains essentially constant.

According to an embodiment, a respiratory signal is monitored in order to determine the beginning and end of the data-redundant breathing phase. The respiratory signal may be monitored via a surrogate signal. A surrogate signal may be signal that defines a surrogate parameter. For example, a surrogate signal may be a signal that is correlated to the breathing state of the subject. For example, the surrogate signal may comprise information about an elevation of the subject's chest. Hence, while breathing typically comprises an expansion and contraction of the lungs (i.e., a three-dimensional object and, thus a change in three-dimensional space) an elevation of the chest (a change in one direction) may be still useful to define the breathing state, since, typically there is a correlation between the elevation of the chest and the whole movement of the lungs/chest. The respiratory signal may be monitored by a strain gauge. The strain gauge may be connected to the subject's chest. For example, the strain gauge may at least partially surround the subject's chest. Accordingly, the CT system may comprise a strain gauge configured to determine the subject's breathing state. The strain gauge may comprise a belt or be part of a belt that is configured to be wrapped around the subject's chest. Hence, the respiratory signal may be monitored by a belt that is wrapped around the subject's chest and that comprises a strain gauge. Additionally or alternatively, the respiratory signal may be monitored via at least one light marker, such as an infrared marker, and a corresponding, camera, such as an infrared camera. In the context of this invention, term "light" is to be understood broadly. It may comprise any kind of electromagnetic signal that can be detected. In particular the term light comprises infrared light. The light marker may be configured to emit a light signal, in particular an infrared signal. Alternatively the light marker may comprise a light reflector, in particular an infrared light reflector. The light marker may be placed on the subject's chest. The camera may be placed at the end of the table. The camera may be configured to receive a light signal from the light marker. The computed tomography system may comprise the light marker and/or the camera. Optionally, the computed tomography system may comprise a light emitter that is configured to emit light such that the light is reflected by the light reflector.

In the context of this invention, the respiratory signal may, for example, be denoted as $\zeta_i$, wherein $i$ denotes a point in time. $\zeta_i$ may in particular denote an elevation of the subject's chest. $\zeta_i$ may also be referred to as breathing state or breathing signal at time point $i$. The derivative of the respiratory signal at time point $i$ may be denoted as $\dot{\zeta}_i$, The derivative of the respiratory signal may also be referred to as the breathing velocity. Based on the monitored respiratory signal, a reference breathing signal $\zeta^{ref}$ may be determined. The reference breathing signal is in particular specific for the subject. Hence, the reference breathing signal may be referred to as subject-specific reference signal. The reference breathing signal may be determined prior to starting the respiration correlated computed tomography scan. The reference breathing signal may be updated based on the respiratory signal monitored during the respiration correlated computed tomography scan. Via the reference breathing signal, a reference breathing cycle may be defined. The reference breathing cycle may be defined analogous to the definition used for generally defining the breathing cycle, e.g. as described herein.

According to an embodiment, a necessary condition for the determination of the beginning of a data-redundant breathing phase is a signal length since the last end-inhalation state having a defined minimum length relative to a reference exhalation duration. Preferably, the condition may be determined to be fulfilled if the condition is or was fulfilled at any point in time during at least one previous or the current respiratory measurement sample during the current breathing cycle. The exhalation duration is in particular the duration from the end-inhalation state to the minimum breathing state or to a breathing state that is above the minimum breathing state by a defined absolute or relative amount. For example, the defined amount may be 5%. The amount may be based on a temporal resolution of the respiration-correlated CT scan. For example, the respiration-correlated CT scan may be configured to take images in steps of a particular percentage of the breathing state amplitude, such as in steps of 5% or 10% of the amplitude. For example, reconstruction bins may be provided that are used to sort CT data based on the breathing states. The reconstruction bins may be defined according to percentage values of the current breathing state relative to the maximum breathing state. For example, one reconstruction bin may be for up 90% to 100%, one for 80% to 90% etc. In the context of this invention, the breathing state amplitude may be seen as the difference between the minimum and the maximum breathing state. Advantageously, the amount may thus be chosen such, that it corresponds to the temporal resolution. The minimum breathing state may be defined to be the breathing state at maximum exhalation. The reference exhalation duration may be based on a subject-specific reference breathing signal and/or an average exhalation duration. Hence, the reference exhalation duration may be the exhalation duration of a reference breathing cycle determined from the reference breathing signal. The average exhalation duration may be determined via the respiration monitoring and by determining the average time that the exhalation duration of the subject takes during the monitoring. Advantageously, the signal length since the last end-inhalation state is thus monitored. Via the condition according to this embodiment it can thus be ensured that the redundancy modulation is not started before a typical exhalation phase of the subject ends. The relation to the reference exhalation may be used to define how sure this condition is with respect to not-missing essential data. The relation may be defined by a pre-factor. The pre-factor may depend on an estimated variance or standard deviation of the subject's exhalation duration. The pre-factor may be pre-set. The pre-factor may be updated dynamically based on the monitored respiratory signal, in particular based on the determined variance or standard deviation. In the context of this invention, the variance or standard deviation is to be understood broadly and encompasses any equivalent measure of data variation. Advantageously, via the pre-factor it may be ensured that the risk of missing essential data can be reduced by adapting the condition accordingly with this pre-factor.

For example, the necessary condition for the determination of the beginning of a data-redundant breathing phase may be defined by the term

$$\exists \zeta_i : \sum_{j=i_{100\%}}^{i} H(\zeta_{j-1} - \zeta_j) \geq 1.1 \times i^{ref}_{5\%exh},$$

or an equivalent term, wherein $i_{100\%}$ is the sample index representing the last state of maximal inhalation (i.e., the beginning of the current breathing cycle), $H(x)$ is the Heaviside step function, $i^{ref}_{5\%exh}$ is the sample index corresponding to the 5% exhalation state, i.e. 5% above the minimum breathing state, in the reference breathing cycle. 1.1 is a pre-factor as described above. This particular pre-factor of 1.1 may be substituted by any other suitable pre-factor. For example, the 1.1 may be replaced by a more general pre-factor $k_1$ in the above term. The 5% exhalation state may be substituted by any other percentage. Optionally, the percentage may be 0%. For example, the 5% in the above term may generally be replaced by x%, x being a free variable defining a percentage value.

According to an embodiment, a necessary condition for the determination of the beginning of a data-redundant breathing phase is the current breathing cycle having gone through a minimum fraction of a reference breathing amplitude during exhalation since the last end-inhalation state. Preferably, the condition may be determined to be fulfilled if the condition is or was fulfilled at any point in time during at least one previous or the current respiratory measurement sample during the current breathing cycle. The fraction of the reference breathing amplitude may be defined by a scaling factor. The scaling factor may be based on an estimated variance or standard deviation of the subject's breathing amplitude. For example, the subject may occasionally inhale deeper than on average. The setting of the scaling factor may be decided based on an expected drift of the measurement. The scaling factor may be pre-set. The scaling factor may be updated dynamically based on the monitored respiratory signal, in particular based on the determined variance or standard deviation. This condition may help to ensure that the redundancy modulation is not applied before the subject is gone through an essentially complete or at least mostly complete exhalation.

For example, the necessary condition for the determination of the beginning of a data-redundant breathing phase may be defined by the term

$$\exists \zeta_i : \zeta_i \leq median(\zeta_{100\%}) - 0.8 \times \Delta\zeta^{ref}$$

or an equivalent term, wherein $median(\zeta_{100\%})$ is the median of all previous samples that represent measurements at end-inhalation, and $\Delta\zeta^{ref}$ is the peak-to-peak amplitude of the reference breathing cycle. 0.8 is a scaling factor as described above. This particular scaling factor of 0.8 may be substituted by any other suitable scaling factor. For example, the 0.8 may be replaced by a more general pre-factor $k_2$ in the above term.

According to an embodiment, a necessary condition for the determination of the beginning of a data-redundant breathing phase is based on the current time-dependent change of the breathing state being low. Preferably, the condition may be determined to be fulfilled if the condition is or was fulfilled at any point in time during at least one previous or the current respiratory measurement sample during the current breathing cycle. Hence, it may be assumed that a low current change of breathing state may indicate the beginning of a data-redundant breathing phase.

According to the invention, a phase space is defined by a coordinate system, with a first axis being the breathing state and a second axis being the time-dependent change of the breathing state, wherein a current polar angle of a point in phase space at the current breathing state and the current time-dependent derivative of the breathing state is defined with respect to the centre of mass of a reference breathing cycle in the phase space, wherein a necessary condition for the determination of the beginning of a data-redundant breathing phase is the current polar angle being greater than or equal to a reference angle based on the reference breathing cycle. Preferably, the condition may be determined to be fulfilled if the condition is or was fulfilled at any point in time during at least one previous or the current respiratory measurement sample during the current breathing cycle.

The time-dependent change of the breathing state may also be referred to as the derivative of the breathing state or the breathing velocity. Via this condition, both a breathing velocity can be monitored to be low and the breathing state can be monitored to be low (i.e. close to the minimum) as well. The reference angle is the angle in the reference breathing cycle at a minimum breathing state or at a breathing state that is above the minimum breathing state by a defined absolute or relative amount. The amount may be defined equivalently as the amount for other conditions as described above.

For example, the necessary condition for the determination of the beginning of a data-redundant breathing phase may be defined by the term

$$\exists \zeta_i : \ \varphi_i \geq \varphi_{5\%exh}^{ref}$$

or an equivalent term, wherein the phase is defined as the polar angle $\varphi_i$ of the point $(\dot{\zeta}_i, \zeta_i)$ with respect to the centre of mass of the reference breathing cycle. Hence, the phase of at least one measurement must have been greater than or equal to the phase at 5% exhalation state in the reference breathing cycle. The 5% exhalation state may be substituted by any other percentage. Optionally, the percentage may be 0%. For example, the 5% in the above term may generally be replaced by y%, y being a free variable defining a percentage value.

Preferably, multiple of the necessary conditions may be applied. Accordingly, the data-redundant breathing phase may be determined to begin only when all of these multiple necessary conditions are fulfilled during one breathing cycle and/or at one couch position.

According to an embodiment, the end of the data-redundant breathing phase is estimated to be reached when the current breathing state exceeds a minimal threshold and/or when a change of the current breathing state exceeds a minimal threshold. For example, an X-ray tube current may be increased again at the end of the data-redundant breathing phase. The minimal threshold may depend on the minimum recorded breathing state of the current breathing cycle. For example, an absolute or relative margin may be added on the minimum recorded breathing state to determine the minimal threshold. According to an embodiment, the end of the data-redundant breathing phase is estimated to be reached when the current breathing state exceeds a defined margin above the minimal breathing state recorded in the current breathing cycle and/or in the current couch position and/or since the last end-inhalation state. For example, the margin may be a percentage value, such as 5%. The margin may be based on a temporal resolution of the respiration-correlated CT scan as explained above with respect to a corresponding amount. The CT system may be configured such that as long as this end condition is fulfilled the beginning of the data-redundant breathing phase may not be triggered. Hence, for example, this may prevent the method to trigger the redundancy modulation at all in some cases, e.g. in cases without a pause at end-exhalation.

According to an embodiment, the X-ray dose is modulated by reducing an X-ray tube current when the redundancy modulation is applied. A reduced x-ray tube current may require a longer acquisition time to achieve a good quality. Accordingly, for parts of the data that are acquired during the redundancy modulation a reconstruction may use data that is acquired over a larger amount of time. In other words, reconstruction bins within this period of redundancy modulation may need to cover a larger time frame. Hence, the temporal resolution is reduced. However, since the breathing state is expected to be mostly constant during this time, motion artifacts can be expected not to occur, due to only very little respiratory motion in this time frame.

According to an embodiment, the X-ray dose is modulated such that the X-ray dose is zero when the redundancy modulation is applied. For example, an X-ray source may be switched off or blocked. Advantageously, this embodiment may reduce the applied X-ray dose even further. Reconstruction bins located within this period may be shifted towards exhalation or inhalation.

The embodiments described herein may be combined with each other unless indicated otherwise. For example, several or all of the necessary conditions may be applied at the same time and/or the application of at least one or several of the necessary conditions may be combined with an embodiment for estimating the end of the data-redundant breathing phase.

According to a further aspect of the invention, a computer program comprising instructions which, when the program is executed by a control unit of a computed tomography system, cause the computed tomography system to carry out the method as described herein, is provided. All features and advantages of the method may be adapted to the computer program and vice versa.

According to a further aspect of the invention, a computer-readable storage medium, in particular non-transient storage medium is provided. The computer-readable storage medium comprises instructions which, when executed by a control unit of a computed tomography system, cause the computed tomography system to carry out the method as described herein. All features and advantages of the method and of the computer program may be adapted to the computer-readable storage medium and vice versa.

According to a further aspect of the invention, a computed tomography system is provided. The computed tomography system comprises a monitoring sensor for monitoring a respiratory signal of a subject, wherein the system is configured to carry out the method as described herein. All features and advantages of the method, of the computer program, and of the computer-readable storage medium may be adapted to the computed tomography system and vice versa. For example, the monitoring sensor may be a strain gauge and or a belt with a strain gauge, in particular as described herein. Additionally or alternatively, the monitoring sensor may be a camera that works together with a light marker as described herein. For example, the computed tomography system may comprise a processing unit that is configured to control and initiate the method steps.

The accompanying drawings illustrate various exemplary embodiments and methods of various aspects of the invention.

Similar elements are designated with the same reference signs in the drawings. In the drawings:

Fig. 1    shows a flow diagram of a method according to an embodiment of the invention;

Fig. 2    shows a computed tomography system according to an embodiment of the invention;

Fig. 3    is a graph of an exemplary respiratory signal, namely a chest elevation in cm, over the time of one breathing cycle;

Fig. 4    is a graph of an exemplary respiratory signal, namely a chest elevation in cm, versus the breathing velocity;

Fig. 5    is a graph of an exemplary respiratory signal, namely a chest elevation in cm, over the time of one breathing cycle;

Fig. 6    is a graph of X-ray dose over time;

Fig. 7    shows two examples of respiratory signals plotted over multiple breathing cycles.

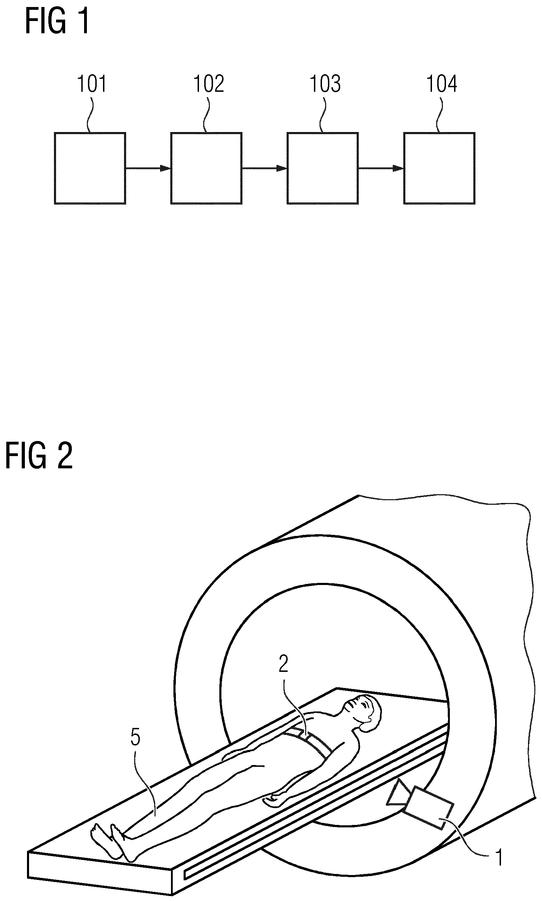

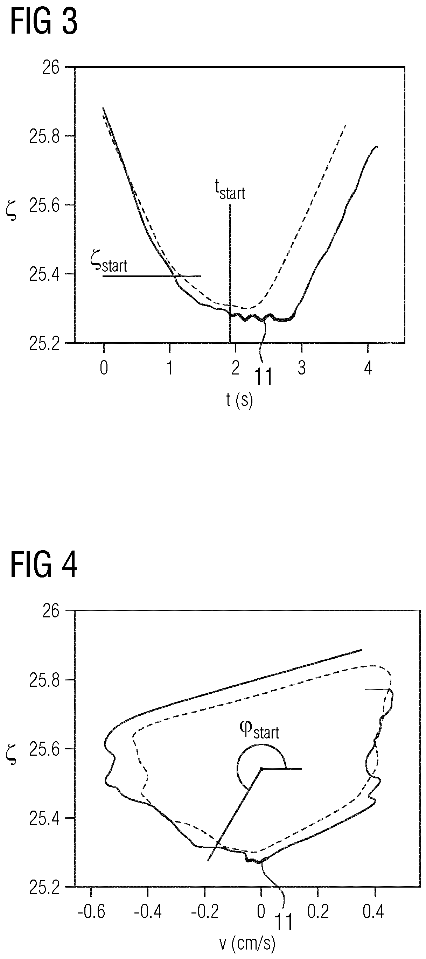

Figure 1 shows a flow diagram of a computer-implemented method for reducing an X-ray dose applied on a subject during a respiration-correlated computed tomography scan according to an embodiment of the invention. In a first step 101, during a beam-on period of the scan, the beginning of a data-redundant breathing phase is determined. For this purpose, a respiratory signal is monitored that can be used to determine the beginning of the data-redundant breathing phase. For example, the respiratory signal may be monitored via an infrared marker 2 being placed on the chest of the subject 5 and a corresponding infrared camera 1. However, the respiratory signal may also be monitored differently e.g. via a strain gauge placed around the subject's 5 chest. The respiratory signal may be a signal that is monitored anyway for the purpose of the respiration-correlated computed tomography scan. For example, a breathing phase may be determined to be data-redundant when it is estimated to provide no significant additional breathing states during the current breathing cycle and/or at the current couch position. This may be done via conditions such as a change of the breathing state over time being estimated to be below a threshold and/or a variance of the breathing state being estimated to remain within a predetermined range. Examples of conditions based on which a data-redundant breathing phase may be determined are given in figures 3 and 4. In a further step 102, during the data-redundant breathing phase, a redundancy modulation is applied to the applied X-ray dose such that the X-ray dose is reduced during the data-redundant breathing phase. The X-ray dose may be modulated by reducing an X-ray tube current when the redundancy modulation is applied. Optionally, the X-ray tube current may be reduced to zero or the X-ray beam may be block such that the X-ray dose applied to the subject 5 is zero when the redundancy modulation is applied. In a further step 103, the end of the data-redundant breathing phase is determined. For this purpose, the monitored respiratory signal may also be used in order to determine the end of the data-redundant breathing phase. In a further step 104, at the end of the data-redundant breathing phase as determined, the scan is continued without the redundancy modulation of the X-ray dose, i.e. by applying the normal X-ray dose.

Figure 2 shows a computed tomography system according to an embodiment of the invention. The system is configured to carry out the method as described with respect to figure 1. The Computed tomography system comprises a monitoring sensor 1 for monitoring a respiratory signal of a subject 5. In this embodiment, the monitoring sensor 1 is an infrared camera 1 that works together with an infrared marker 2 on the subject's 5 chest.

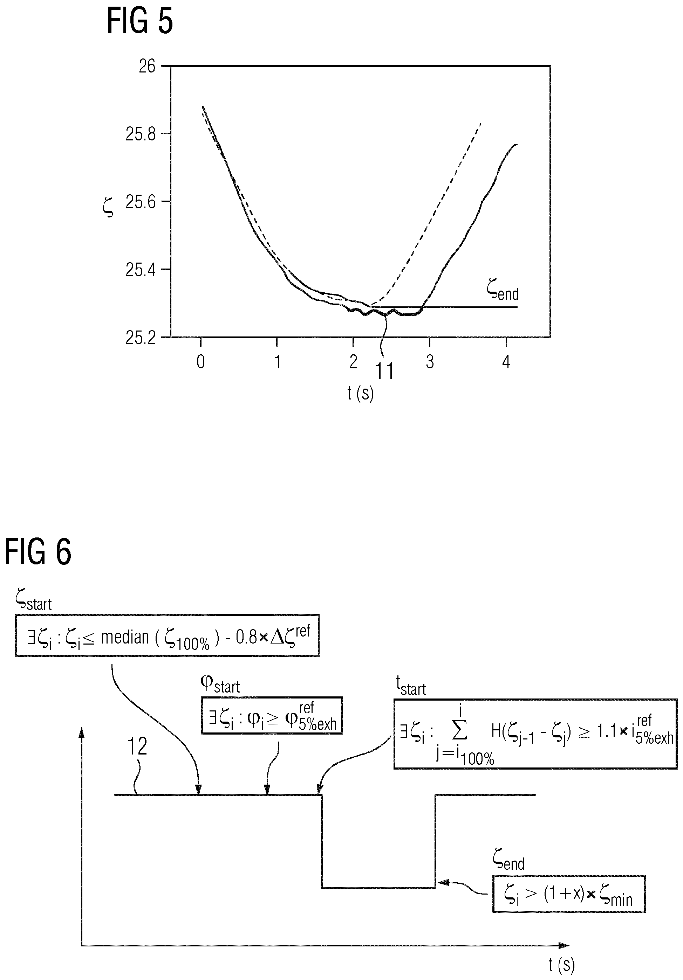

Figures 3 to 5 illustrate an exemplary breathing cycle and the application of conditions for determining the beginning (figures 3 and 4) and end (figure 5) of a data-redundant breathing phase. On the vertical axis of the plots the respiratory signal is plotted, which is a c. The absolute values given here are arbitrary based on the monitoring setup.

Relevant are the relevant values between the different breathing states. The horizontal axis of figures 3 and 5 shows the time in seconds. The horizontal axis of figure 4 shows the breathing velocity in cm/s. Generally, the respiratory signal (also referred to as breathing state or breathing signal), corresponding to an elevation of a subject's 5 chest, is denoted as $\zeta_i$, wherein $i$ denotes a point in time. The derivative of the respiratory signal (also referred to as breathing velocity) at time point $i$ is denoted as $\dot{\zeta}_i$. The respiratory signal $\zeta_i$ (figures 3 and 5) and the breathing velocity $\dot{\zeta}_i$ (figure 4) are shown as continuous line. Based on the monitored respiratory signal, a reference breathing signal is denoted $\zeta^{ref}$. The reference breathing signal $\zeta^{ref}$ is shown as dashed line.

In this embodiment, there are three necessary conditions for the determination of the beginning of a data-redundant breathing phase. These conditions are a time criterion ($t_{start}$, see figure 3), an amplitude criterion ($\zeta_{start}$, see figure 3), and a phase criterion ($\varphi_{start}$, see figure 4). Furthermore, there is an end criterion ($\zeta_{end}$, see figure 5) that determines the end of the data-redundant breathing phase.

The time criterion is defined by the term

$$\exists \zeta_i : \sum_{j=i_{100\%}}^{i} H\left(\zeta_{j-1} - \zeta_j\right) \geq 1.1 \times i_{5\%exh}^{ref} \,,$$

wherein $i_{100\%}$ is the sample index representing the last state of maximal inhalation (i.e., the beginning of the current breathing cycle), $H(x)$ is the Heaviside step function, $i_{5\%exh}^{ref}$ is the sample index corresponding to the 5% exhalation state, i.e. 5% above the minimum breathing state, in the reference breathing cycle. This time criterion is marked in figure 3 with a vertical line that is named "$t_{start}$". The time criterion is fulfilled as soon as the time (tracked on the horizontal axis) is beyond this line.

The amplitude criterion is defined by the term

$$\exists \zeta_i : \zeta_i \leq median(\zeta_{100\%}) - 0.8 \times \Delta\zeta^{ref}$$

wherein $median(\zeta_{100\%})$ is the median of all previous samples that represent measurements at end-inhalation, and $\Delta\zeta^{ref}$ is the peak-to-peak amplitude of the reference breathing cycle. This amplitude criterion is marked in figure 3 with a horizontal line that is named "$\zeta_{start}$". The amplitude criterion is fulfilled as soon as the respiratory signal $\zeta$ drops below this line.

The phase criterion is defined by the term

$$\exists \zeta_i : \varphi_i \geq \varphi_{5\%exh}^{ref} \,,$$

wherein the phase is defined as the polar angle $\varphi_i$ of the point $(\dot{\zeta}_i, \zeta_i)$ with respect to the centre of mass of the reference breathing cycle. Hence, the phase of at least one measurement must have been greater than or equal to the phase at 5% exhalation state in the reference breathing cycle. This phase criterion is marked in figure 4 with an angle line that is named "$\varphi_{start}$" and that has its origin in the centre of mass of the reference breathing cycle (dashed line). The phase criterion is fulfilled as soon as the angle of the point $(\dot{\zeta}_i, \zeta_i)$, that moves counter-clockwise, crosses this angle line.

If at any point in time each of the three criteria has been fulfilled during at least one previous (or the current) measurement sample during the current breathing cycle, the redundancy modulation is applied. The duration of the redundancy modulation is marked 11. In this example, the critical criterion, i.e. the criterion fulfilled last, is the time criterion ($\zeta_{start}$). However, this may differ depending on properties of the individual subject's 5 breathing cycle.

The redundancy modulation is stopped as soon as the end criterion is fulfilled. Namely the data-redundant breathing phase and thus the redundancy modulation ends as soon as the breathing signal exceeds a certain margin above the minimal breathing signal of the current cycle. In figure 5 it can be seen that the minimum breathing signal and thus the end criterion ($\zeta_{end}$) changes with time during the exhalation phase and is mostly constant during the data redundant breathing phase. When inhalation begins (i.e. the respiratory signal exhibits an incline) the end criterion also remains constant. The incline of the respiratory signal triggers the end criterion $\zeta_{end}$ and, thus, the end of the redundancy modulation.

Figure 6 illustrates a rough representation of the amount 12 of the X-ray dose that is applied before, during, and after a data-redundant breathing phase. At first a normal, i.e. higher X-ray dose is applied. Then, consecutively, the amplitude criterion ($\zeta_{start}$), the phase criterion ($\varphi_{start}$), and the time criterion ($t_{start}$) are fulfilled. As soon as all three criteria ($t_{start}$ being the last) are fulfilled, the redundancy modulation is applied by reducing the X-ray dose. As soon as the end criterion ($\zeta_{end}$) is fulfilled, the redundancy modulation is stopped, and the original X-ray dose is applied again.

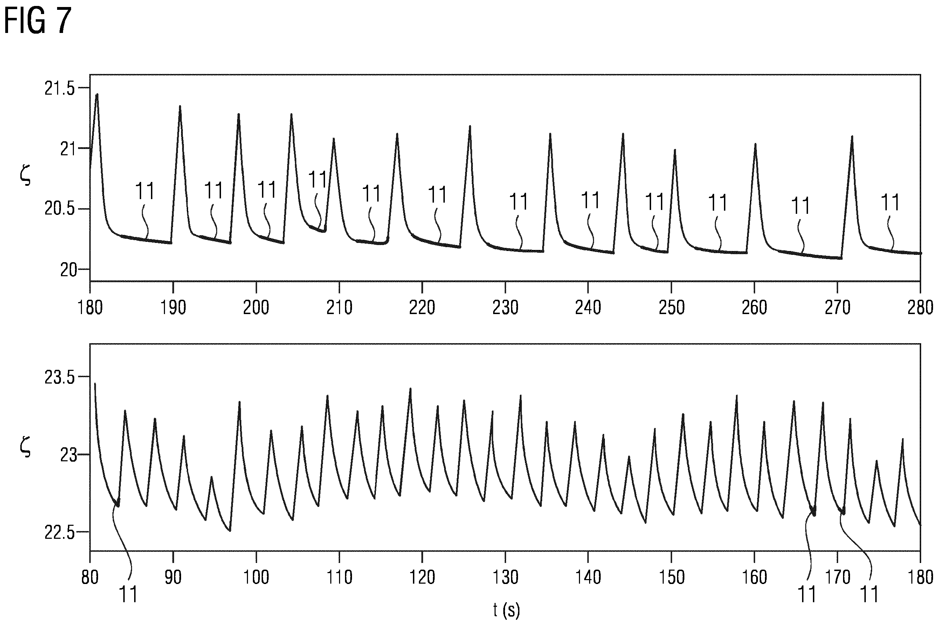

Figure 7 shows two examples of respiratory signals plotted over multiple breathing cycles. The vertical axis represents the respiratory signal $\zeta$ and the horizontal axis represents the time t (in seconds). In the upper example there are long breathing pauses that occur after each exhalation. Correspondingly there are long data-redundant phases (marked with markings 11) during which the redundancy modulation is applied. Thus, in this upper example, a considerable amount of X-ray dose can be reduced. Up to 50% of the X-ray dose applied to the patient may be saved without reducing data completeness to reconstruct a full 4D-CT data set. In the lower example, there are only a few short breathing pauses that occur after each exhalation. Correspondingly there are only a few short data-redundant phases (marked with markings 11) during which the redundancy modulation is applied. In this lower example, only little X-ray dose may be saved.

**Claims**

1. A computer-implemented method for reducing an X-ray dose (12) applied on a subject (5) during a respiration-correlated computed tomography scan, the method comprising the following steps:

   (a) during a beam-on period of the scan, determining the beginning of a data-redundant breathing phase;
   (b) during the data-redundant breathing phase, applying a redundancy modulation to the applied X-ray dose (12) such that the X-ray dose is reduced during the data-redundant breathing phase;
   (c) determining the end of the data-redundant breathing phase;
   (d) at the end of the data-redundant breathing phase, continuing the scan without the redundancy modulation of the X-ray dose;
   **characterized in that** a phase space is defined by a coordinate system, with a first axis being the breathing state and a second axis being the time-dependent change of the breathing state,
   wherein a current polar angle of a point in phase space at the current breathing state and the current time-dependent derivative of the breathing state is defined with respect to the centre of mass of a reference breathing cycle in the phase space,
   wherein a necessary condition for the determination of the beginning of a data-redundant breathing phase is the current polar angle being greater than or equal to a reference angle based on the reference breathing cycle, wherein the reference angle is the angle in the reference breathing cycle at a minimum breathing state or at a breathing state that is above the minimum breathing state by a defined absolute or relative amount.

2. The method according to claim 1,
   wherein a breathing phase is determined to be data-redundant when it is estimated to provide no significant additional breathing states during the current breathing cycle and/or at the current couch position.

3. The method according to any one of the preceding claims, wherein a breathing phase is determined to be data-redundant, when a change of the breathing state over time is estimated to be below a threshold and/or when a variance of the breathing state is estimated to remain within a predetermined range.

4. The method according to any one of the preceding claims, wherein a necessary condition for the determination of the beginning of a data-redundant breathing phase is a signal length since the last end-inhalation state having a defined minimum length relative to a reference exhalation duration.

5. The method according to any one of the preceding claims, wherein a necessary condition for the determination of the beginning of a data-redundant breathing phase is the current breathing cycle having gone through a minimum fraction of a reference breathing amplitude during exhalation since the last end-inhalation state.

6. The method according to any one of the preceding claims, wherein a necessary condition for the determination of the beginning of a data-redundant breathing phase is based on the current time-dependent change of the breathing state being low.

7. The method according to any one of the preceding claims, wherein the condition is determined to be fulfilled if the condition is or was fulfilled at any point in time during at least one previous or the current respiratory measurement sample during the current breathing cycle.

8. The method according to any one of the preceding claims, wherein the end of the data-redundant breathing phase is estimated to be reached when the current breathing state exceeds a minimal threshold and/or when a change of the current breathing state exceeds a minimal threshold.

9. The method according to any one of the preceding claims, wherein the end of the data-redundant breathing phase is estimated to be reached when the current breathing state exceeds a defined margin above the minimal breathing state recorded in the current breathing cycle and/or in the current couch position and/or since the last end-inhalation state.

10. The method according to any one of the preceding claims, wherein the X-ray dose is modulated by reducing an X-ray tube current when the redundancy modulation is applied.

11. The method according to any one of the preceding claims, wherein the X-ray dose is modulated such that the X-ray dose is zero when the redundancy modulation is applied.

12. The method according to any one of the preceding claims, wherein a respiratory signal is monitored in order to determine the beginning and end of the data-redundant breathing phase.

13. A computer program comprising instructions which, when the program is executed by a control unit of the computed tomography system of claim 15, cause the computed tomography system to carry out the method of any one of the preceding claims.

14. A computer-readable storage medium, in particular non-transient storage medium, comprising instructions which, when executed by a control unit of the computed tomography system of claim 15, cause the computed tomography system to carry out the method of any one of claims 1 to 12.

15. A computed tomography system,

comprising a monitoring sensor for monitoring a respiratory signal of a subject (5),
wherein the system is configured to carry out the method according to any one of claims 1 to 12.

**Patentansprüche**

1. Computerimplementiertes Verfahren zum Reduzieren einer Röntgendosis (12), die während eines atemkorrelierten Computertomographiescans auf ein Subjekt (5) angewendet wird, wobei das Verfahren die folgenden Schritte umfasst:

(a) Bestimmen des Beginns einer datenredundanten Atmungsphase während einer Beam-On-Periode des Scans;
(b) Anwenden einer Redundanzmodulation auf die angewandte Röntgendosis (12) während der datenredundanten Atmungsphase, so dass die Röntgendosis während der datenredundanten Atmungsphase reduziert wird;
(c) Bestimmen des Endes der datenredundanten Atmungsphase;
(d) Fortsetzen des Scans am Ende der datenredundanten Atmungsphase ohne Redundanzmodulation der Röntgendosis; **dadurch gekennzeichnet, dass** ein Phasenraum durch ein Koordinatensystem definiert ist, wobei eine erste Achse der Atmungszustand ist und eine zweite Achse die zeitabhängige Änderung des Atmungszustands ist,
wobei ein aktueller Polarwinkel eines Punkts im Phasenraum bei dem aktuellen Atmungszustand und der aktuellen zeitabhängigen Ableitung des Atmungszustands in Bezug auf den Massenschwerpunkt eines Referenzatmungszyklus in dem Phasenraum definiert ist,
wobei eine notwendige Bedingung für die Bestimmung des Beginns einer datenredundanten Atmungsphase darin liegt, dass der aktuelle Polarwinkel größer als oder gleich einem Referenzwinkel ist, basierend auf dem Referenzatmungszyklus,
wobei der Referenzwinkel der Winkel in dem Referenzatmungszyklus bei einem minimalen Atmungszustand oder bei einem Atmungszustand ist, der um einen definierten absoluten oder relativen Betrag über dem minimalen Atmungszustand liegt.

2. Verfahren nach Anspruch 1,
wobei eine Atmungsphase als datenredundant bestimmt wird, wenn geschätzt wird, dass sie keine signifikanten zusätzlichen Atmungszustände während des aktuellen Atmungszyklus und/oder an der aktuellen Liegeposition bereitstellt.

3. Verfahren nach einem der vorhergehenden Ansprüche,

wobei eine Atmungsphase als datenredundant bestimmt wird, wenn geschätzt wird, dass eine Änderung des Atmungszustands über die Zeit unter einer Schwelle liegt, und/oder wenn geschätzt wird, dass eine Varianz des Atmungszustands innerhalb eines vorbestimmten Bereichs bleibt.

4. Verfahren nach einem der vorhergehenden Ansprüche,
wobei eine notwendige Bedingung für die Bestimmung des Beginns einer datenredundanten Atmungsphase eine Signallänge seit dem letzten Endinhalationszustand mit einer definierten Mindestlänge relativ zu einer Referenzexhalationsdauer ist.

5. Verfahren nach einem der vorhergehenden Ansprüche,
wobei eine notwendige Bedingung für die Bestimmung des Beginns einer datenredundanten Atmungsphase ist, dass der aktuelle Atmungszyklus während der Exhalation seit dem letzten Endinhalationszustand einen Mindestbruchteil einer Referenzatmungsamplitude durchlaufen hat.

6. Verfahren nach einem der vorhergehenden Ansprüche,
wobei eine notwendige Bedingung für die Bestimmung des Beginns einer datenredundanten Atmungsphase darauf basiert, dass die aktuelle zeitabhängige Änderung des Atmungszustands gering ist.

7. Verfahren nach einem der vorhergehenden Ansprüche,
wobei bestimmt wird, dass die Bedingung erfüllt ist, wenn die Bedingung zu einem beliebigen Zeitpunkt während mindestens einer vorherigen oder der aktuellen respiratorischen Messabtastung während des aktuellen Atmungszyklus erfüllt ist oder war.

8. Verfahren nach einem der vorhergehenden Ansprüche,
wobei geschätzt wird, dass das Ende der datenredundanten Atmungsphase erreicht ist, wenn der aktuelle Atmungszustand eine minimale Schwelle überschreitet und/oder wenn eine Änderung des aktuellen Atmungszustands eine minimale Schwelle überschreitet.

9. Verfahren nach einem der vorhergehenden Ansprüche,
wobei geschätzt wird, dass das Ende der datenredundanten Atmungsphase erreicht ist, wenn der aktuelle Atmungszustand eine definierte Spanne über dem minimalen Atmungszustand überschreitet, der in dem aktuellen Atmungszyklus und/oder in der aktuellen Liegeposition und/oder seit dem letzten Endinhalationszustand aufgezeichnet worden ist.

10. Verfahren nach einem der vorhergehenden Ansprüche,
wobei die Röntgendosis durch Reduzieren eines Röntgenröhrenstroms moduliert wird, wenn die Redundanzmodulation angewendet wird.

11. Verfahren nach einem der vorhergehenden Ansprüche,
wobei die Röntgendosis derart moduliert wird, dass die Röntgendosis null ist, wenn die Redundanzmodulation angewendet wird.

12. Verfahren nach einem der vorhergehenden Ansprüche,
wobei ein respiratorisches Signal überwacht wird, um den Beginn und das Ende der datenredundanten Atmungsphase zu bestimmen.

13. Computerprogramm, umfassend Anweisungen, die bei Ausführung des Programms durch eine Steuereinheit des Computertomographiesystems nach Anspruch 15 bewirken, dass das Computertomographiesystem das Verfahren nach einem der vorhergehenden Ansprüche ausführt.

14. Computerlesbares Speichermedium, insbesondere nichtflüchtiges Speichermedium, umfassend Anweisungen, die bei Ausführung durch eine Steuereinheit des Computertomographiesystems nach Anspruch 15 bewirken, dass das Computertomographiesystem das Verfahren nach einem der Ansprüche 1 bis 12 durchführt.

15. Computertomographiesystem,

umfassend einen Überwachungssensor zum Überwachen eines respiratorischen Signals eines Subjekts (5), wobei das System dazu ausgelegt ist, das Verfahren nach einem der Ansprüche 1 bis 12 durchzuführen.

**Revendications**

1. Un procédé mis en œuvre par ordinateur de réduction de la dose (12) de rayons X appliquée à un sujet (5) pendant un scan de tomodensitométrie informatisée corrélé à la respiration, le procédé comprenant les stades suivants :

   (a) pendant une durée d'incidence du faisceau du scan, déterminer le début d'une phase respiratoire redondante en données ;
   (b) pendant la phase respiratoire redondante en données, appliquer une modulation de redondance à la dose (12) de rayons X appliquée, de manière à réduire la dose de rayons X pendant la phase respiratoire redondante en données ;
   (c) déterminer la fin de la phase respiratoire redondante en données ;
   (d) à la fin de la phase respiratoire redondante en données, continuer le scan sans la modulation de redondance de la dose de rayons X ;
   **caractérisé en ce qu'**un espace de phase est défini par un système de coordonnées avec un premier axe, qui est l'état respiratoire et un deuxième axe, qui est la variation en fonction du temps de l'état respiratoire,
   dans lequel un angle polaire en cours d'un point dans l'espace de phase à l'état respiratoire en cours et la dérivée en cours en fonction du temps de l'état respiratoire est défini par rapport au centre de masse d'un cycle respiratoire de référence dans l'espace de phase,
   dans lequel une condition nécessaire pour la détermination du début d'une phase respiratoire redondante en données est que l'angle polaire en cours soit supérieur ou égal à un angle de référence reposant sur le cycle respiratoire de référence,
   dans lequel l'angle de référence est l'angle dans le cycle respiratoire de référence à un état respiratoire minimum ou à un état respiratoire, qui est au-dessus de l'état respiratoire minimum d'une quantité absolue ou relative définie.

2. Le procédé suivant la revendication 1,
   dans lequel une phase respiratoire est déterminée comme étant redondante en données lorsqu'elle est estimée comme ne donnant pas d'état respiratoire supplémentaire significatif pendant le cycle respiratoire en cours et/ou à la position couchée en cours.

3. Le procédé suivant l'une quelconque des revendications précédentes,
   dans lequel une phase respiratoire est déterminée comme étant redondante en données lorsqu'on estime qu'un changement d'un état respiratoire en fonction du temps est en-dessous d'un seuil et/ou lorsqu'on estime qu'une variance de l'état respiratoire reste dans une plage déterminée à l'avance.

4. Le procédé suivant l'une quelconque des revendications précédentes,
   dans lequel une condition nécessaire à la détermination du début d'une phase respiratoire redondante en données est une longueur de signal, depuis le dernier état d'inhalation de fin, ayant une longueur minimum définie par rapport à une durée d'exhalation de référence.

5. Le procédé suivant l'une quelconque des revendications précédentes,
   dans lequel une condition nécessaire à la détermination du début d'une phase respiratoire redondante en données est que le cycle respiratoire en cours soit passé par une fraction minimum d'une amplitude respiratoire de référence pendant une exhalation depuis le dernier état d'inhalation de fin.

6. Le procédé suivant l'une quelconque des revendications précédentes,
   dans lequel une condition nécessaire à la détermination du début d'une phase respiratoire redondante en données repose sur le fait que le changement de l'état respiratoire en fonction du temps est petit.

7. Le procédé de l'une quelconque des revendications précédentes,
   dans lequel la condition est déterminée comme étant satisfaite, si la condition est ou était satisfaite à n'importe quel point dans le temps pendant au moins un échantillon de mesure respiratoire en cours ou un échantillon de mesure respiratoire précédent pendant le cycle respiratoire en cours.

8. Le procédé suivant l'une quelconque des revendications précédentes,
   dans lequel il est estimé que la fin de la phase respiratoire redondante en données est atteinte lorsque l'état respiratoire en cours dépasse un seuil minimum et/ou lorsqu'un changement de l'état respiratoire en cours dépasse un seuil minimum.

**9.** Le procédé suivant l'une quelconque des revendications précédentes,
dans lequel on estime que la fin de la phase respiratoire redondante est atteinte lorsque l'état respiratoire en cours dépasse une marge définie au-dessus de l'état respiratoire minimum enregistré dans le cycle respiratoire en cours et/ou dans la position couchée en cours et/ou depuis le dernier état d'inhalation de fin.

**10.** Le procédé de l'une quelconque des revendications précédentes,
dans lequel la dose de rayons X est modulée en réduisant un courant de tube de rayons X lorsque la modulation de redondance est appliquée.

**11.** Le procédé de l'une quelconque des revendications précédentes,
dans lequel la dose de rayons X est modulée de manière à ce que la dose de rayons X soit de zéro lorsque la modulation de redondance est appliquée.

**12.** Le procédé de l'une quelconque des revendications précédentes,
dans lequel un signal respiratoire est surveillé afin de déterminer le début et la fin de la phase respiratoire redondante en données.

**13.** Un programme d'ordinateur comprenant des instructions qui, lorsque le programme est exécuté par une unité de commande du système de tomodensitométrie informatisée de la revendication 15, font que le système de tomodensitométrie informatisée exécute le procédé suivant l'une quelconque des revendications précédentes.

**14.** Un support de mémoire déchiffrable par ordinateur, en particulier un support de mémoire non transitoire, comprenant des instructions qui, lorsqu'elles sont exécutées par une unité de commande du système de tomodensitométrie informatisée, font que le système de tomodensitométrie informatisée de la revendication 15 exécute le procédé de l'une quelconque des revendications 1 à 12.

**15.** Un système de tomodensitométrie informatisée,

comprenant un capteur de surveillance pour surveiller un signal respiratoire d'un sujet (5),
dans lequel le système est configuré pour exécuter un procédé suivant l'une des revendications 1 à 12.

FIG 1

FIG 2

## FIG 3

## FIG 4

## FIG 5

## FIG 6

$\zeta_{start}$

$$\exists \zeta_i : \zeta_i \leq \text{median} \, (\zeta_{100\%}) - 0.8 \times \Delta\zeta^{ref}$$

$\varphi_{start}$

$$\exists \zeta_i : \varphi_i \geq \varphi_{5\%exh}^{ref}$$

$t_{start}$

$$\exists \zeta_i : \sum_{j=i_{100\%}}^{i} H(\zeta_{j-1} - \zeta_j) \geq 1.1 \times i_{5\%exh}^{ref}$$

$\zeta_{end}$

$$\zeta_i > (1+x) \times \zeta_{min}$$

## FIG 7

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102011079496 A1

**Non-patent literature cited in the description**

- **R. WERNER** ; **T. SENTKER** ; **F. MADESTA** ; **T. GAUER** ; **C. HOFMANN**. Intelligent 4D CT sequence scanning (i4DCT): Concept and performance Evaluation. *Med. Phys.*, August 2019, vol. 46 (8), 3462-3474